(19) Europäisches Patentamt
European Patent Office
Office européen des brevets

(11) EP 2 746 281 B1

(12) EUROPÄISCHE PATENTSCHRIFT

(45) Veröffentlichungstag und Bekanntmachung des Hinweises auf die Patenterteilung:
**26.04.2017 Patentblatt 2017/17**

(51) Int Cl.:
***C07D 471/04*** (2006.01) ***C07D 519/00*** (2006.01)
***A61K 31/437*** (2006.01) ***A61P 35/00*** (2006.01)

(21) Anmeldenummer: **14000623.0**

(22) Anmeldetag: **09.01.2012**

(54) **7-Azaindolderivate**

7-azaindole derivatives

Dérivés de 7-azaindole

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2011 DE 102011009961**

(43) Veröffentlichungstag der Anmeldung:
**25.06.2014 Patentblatt 2014/26**

(62) Dokumentnummer(n) der früheren Anmeldung(en) nach Art. 76 EPÜ:
**12701437.1 / 2 670 748**

(73) Patentinhaber: **Merck Patent GmbH**
**64293 Darmstadt (DE)**

(72) Erfinder:
- **Dorsch, Dieter**
  **64372 Ober-Ramstadt (DE)**
- **Sirrenberg, Christian**
  **64285 Darmstadt (DE)**
- **Mueller, Thomas J.J.**
  **40591 Duesseldorf (DE)**
- **Merkul, Eugen**
  **40225 Duesseldorf (DE)**
- **Amatunu Karapetyan, Gnuni**
  **49152 BAD Essen (DE)**

(56) Entgegenhaltungen:
**WO-A1-2005/047266** **WO-A1-2005/054232**
**WO-A1-2006/050076** **WO-A1-2006/063167**
**WO-A1-2006/130673** **WO-A1-2009/054941**
**WO-A1-2010/051781** **WO-A1-2010/127754**
**WO-A1-2012/000595** **WO-A2-2007/149427**
**DE-A1-102006 012 617** **DE-A1-102007 028 515**
**DE-A1-102008 025 751** **DE-A1-102008 031 517**
**DE-A1-102008 038 221** **DE-A1-102009 019 962**
**US-A1- 2007 203 142** **US-A1- 2009 215 750**
**US-B2- 7 507 826** **US-B2- 7 528 147**

- **DATABASE WPI Week 200745 Thomson Scientific, London, GB; AN 2007-462575 XP002722320, -& JP 2007 099640 A (TSUMURA & CO) 19. April 2007 (2007-04-19)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die Erfindung betrifft Verbindungen der Formel I

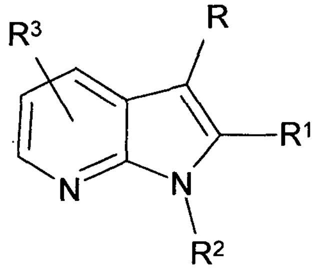

I

worin

R unsubstituiertes oder ein- oder zweifach durch $R^5$ substituiertes 2,7-Naphthyridin-4-yl,

$R^1$ H oder A',

$R^2$ H, A' oder $-[C(R^6)_2]_n$-Ar,

$R^3$ H, A, Hal, CN, $-[C(R^6)_2]_n$-Ar, $-[C(R^6)_2]_n$-Het, $-[C(R^6)_2]_n$-CyC, $OR^6$ oder $N(R^6)_2$,

$R^5$ A, Hal, CN, $-[C(R^6)_2]_n$-Ar, $-[C(R^6)_2]_n$-Het, $-[C(R^6)_2]_n$-Cyc, OCyc, OHet', $OR^6$, $N(R^6)_2$, $SO_2A$, $SO_2Ar$ oder =O,

$R^6$ H oder A',

A unverzweigtes oder verzweigtes Alkyl mit 1-6 C-Atomen, worin eine oder zwei $CH_2$-Gruppen durch O-, N und/oder S-Atome und/oder durch -CH=CH-Gruppen und/oder auch 1-7 H-Atome durch F ersetzt sein können,

A' unverzweigtes oder verzweigtes Alkyl mit 1-4 C-Atomen oder Cyc,

Cyc Cycloalkyl mit 3, 4, 5, 6 oder 7 C-Atomen,

Ar unsubstituiertes oder ein-, zwei- oder dreifach durch Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2N(R^6)_2$ und/oder $S(O)_nA$ substituiertes Phenyl,

Het einen unsubstituierten oder ein- oder zweifach durch Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2NR^6$ und/oder $S(O)_nA$ substituierten ein- oder zweikernigen gesättigten, ungesättigten oder aromatischen Heterocyclus mit 1 bis 4 N-, O- und/oder S-Atomen,

Het' einen unsubstituierten oder ein- oder zweifach durch A und/oder =O substituierten einkernigen gesättigten Heterocyclus mit 1 oder 2 N-, O- und/oder S-Atomen,

Hal F, Cl, Br oder I,

n 0, 1 oder 2

bedeuten,
sowie ihre pharmazeutisch verwendbaren Salze, Tautomere und Stereoisomere, einschließlich deren Mischungen in allen Verhältnissen.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze, Tautomere und Stereoisomere, einschließlich deren Mischungen in allen Verhältnissen, bei guter Verträglichkeit sehr wertvolle pharmakologische Eigenschaften besitzen.

Insbesondere zeigen sie eine inhibierende Wirkung der Zellproliferation/ Zellvitalität als Antagonisten oder

Agonisten. Die erfindungsgemäßen Verbindungen können daher zur Bekämpfung und/oder Behandlung von Tumoren, Tumorwachstum und/oder Tumormetastasen verwendet werden. Die antiproliferative Wirkung kann in einem Proliferationsassay/Vitalitätsassay getestet werden.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).

Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch zur Verwendung in einem Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt. Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, perianastomotische Prothesenrestenose, Restenose nach Angioplastie oder Stent-Platzierung und dergleichen.

Die Verbindungen der Formel I, wirken auch als Regulatoren, Modulatoren oder Inhibitoren von Proteinkinasen, insbesondere des Typs Serin/Threonin-Kinase, zu denen unter anderem die Phosphoinositid-abhängige Kinase 1 (PDK 1) gehören. Eine gewisse Wirkung zeigen die erfindungsgemäßen Verbindungen bei der Inhibierung der Serin/Threonin-Kinasen PDK1, IKKε und TBK1.

PDK1 phosphoryliert und aktiviert eine Untergruppe der AGC Proteinkinasen-Familie, umfassend PKB, SGK, S6K und PKC Isoformen. Diese Kinasen sind an dem PI3K Signalübertragungsweg beteiligt und kontrollieren grundlegende zelluläre Funktionen wie Überleben, Wachstum und Differenzierung. PDK1 ist somit ein bedeutender Regulator diverser metabolischer, proliferativer und Lebenserhaltungs-Effekte.

Die erfindungsgemäßen Verbindungen zeigen auch TGFβ-Rezeptor I-Kinase inhibierende Eigenschaften. Eine Reihe von Erkrankungen wurden mit der Überproduktion von TGF-β1 in Zusammenhang gebracht. Inhibitoren des intrazellulären TGF-β-Signalwegs sind geeignete Behandlungen für fibroproliferative Erkrankungen. Fibroproliferative Erkrankungen umfassen spezifisch Nierenstörungen, die mit einer unregulierten TGF-β-Aktivität einhergehen, und starke Fibrose, einschließlich Glomerulonephritis (GN), wie mesangiale proliferative GN, Immun-GN und Halbmond-GN. Andere Nierenzustände umfassen diabetische Nephropathie, renale interstitielle Fibrose, renale Fibrose bei Transplantat-Patienten, die Cyclosporin erhalten, und mit HIV einhergehende Nephropathie. Collagen-Gefäßstörungen umfassen progressive systemische Sklerose, Polymyositis, Sklerodermie, Dermatomyositis, eosinophile Fascitis, Morphea oder solche Störungen, die mit dem Vorkommen des Raynaud-Syndroms einhergehen. Lungenfibrosen, die durch eine übermäßige TGF-β-Aktivität verursacht werden, umfassen das Atemstörungssyndrom bei Erwachsenen, idiopathische Lungenfibrose und interstitielle Lungenfibrose, die oft mit Autoimmunstörungen einhergeht, wie systemischer Lupus erythematodes und Sklerodermie, chemischer Kontakt oder Allergien. Eine weitere Autoimmunstörung, die mit fibroproliferativen Eigenschaften einhergeht, ist rheumatoide Arthritis.

Augenerkrankungen, die mit einem fibroproliferativen Zustand einhergehen, umfassen eine proliferative Vitreoretinopathie, die bei einer Wiederbefestigungsoperation der Retina vorkommt, Katarakt-Extraktion mit einer intraokularen Linsenimplantation, und Post-Glaukom-Drainagenoperation, und gehen mit einer TGF-β1-Überproduktion einher.

In der WO 2005/095400 A1 sind andere Azaindol-Derivate als Proteinkinaseinhibitoren beschrieben.
In der WO 2008/079988 A2 sind Chinazolinderivate als PDK1-Inhibitoren zur Krebsbekämpfung beschrieben.
In der WO 2008/112217 A1 sind Benzonaphthyridinderivate als PDK1-Inhibitoren zur Krebsbekämpfung beschrieben.
Pyridinonylderivate kennt man als PDK1-Inhibitoren zur Krebsbekämpfung aus der WO 2008/005457.
Pyrrolo-pyridin-kinase-modulatoren zur Krebsbekämpfung sind in WO 2008/124849 beschrieben.
In der WO 2006/106326 A1 und WO 2008/156726 A1 sind andere heterocyclische Verbindungen als PDK1-Inhibitoren zur Krebsbekämpfung beschrieben.
In der WO 2009/054941 A1 sind Pyrrolopyridin-derivate als PDK1-Inhibitoren zur Krebsbekämpfung beschrieben.
JP 2007-099640 A offenbart Pyrrolo[2,3-b]pyridine mit substituiertem Chinoxalinon-Rest in 3-Position als VEGF-Inhibitoren.
US 2007/0203142 A1 offenbart Pyrrolo[2,3-b]pyridine mit substituiertem Pyrazin- oder Triazin-Rest in 3-Position als JAK-Inhibitoren.
WO 2012/000595 A1 beschreibt 1,8-Naphthyridin-Verbindungen, die in 4-Position einen Aryl- oder Heteroaryl-Rest tragen, wobei der Heteroaryl-Rest u.a. auch ein Pyrrolo[2,3-b]pyridin-Substituent sein kann; die 1,8-Naphthyridin-Verbindungen werden als TGF-beta Inhibitoren beschrieben.

IKKε und TBK1 sind Serin/Threonin Kinasen die hohe Homologien untereinander sowie zu anderen IkB-Kinasen aufweisen. Beide Kinasen spielen eine integrale Role für das angebore, immanente Immunsystem. Dopplesträngige RNA-Viren werden durch die Toll-like Rezeptoren 3 und 4, sowie die RNA-Helicasen RIG-I and MDA-5 erkannt und führen zu einer Aktivierung der TRIF-TBK1/IKKε-IRF3 Signalkaskade, was zu einer Typ I Interferon-Antwort führt.

Boehm und Kollegen beschrieben 2007 IKKε als ein neuartiges Brustkrebsonkogen [J.S. Boehm et al., Cell 129, 1065-1079, 2007]. 354 Kinasen wurden auf Ihre Fähigkeit hin untersucht gemeinschaftlich mit einer aktivierten Form der MAPK Kinase Mek, den Ras -transformierenden Phänotyp zu rekapitulieren. IKKε wurde hierbei als ein kooperatives Onkogen identifiziert. Darüberhinaus konnten die Autoren zeigen, dass IKBKE in zahlreichen Brustkrebszellinien und Tumorproben amplifiziert und überexpremiert vorliegt. Die Verminderung der Genexpression mittels RNA interference in Brustkrebszellen induziert Apoptosis und beeinträchtigt deren Proliferation. Eddy und Kollegen kamen 2005 zu ähnlichen Befunden, was die Bedeutung von IKKε in Brustkrebserkrankungen unterstreicht [S.F.Eddy et al., Cancer Res. 2005; 65 (24), 11375-11383].

Über einen protumorigenen Effekt von TBK1 wurde erstmals 2006 berichtet. Korherr und Kollegen identifizierten in einem Screening einer 251000 cDNA umfassenden Genbibliothek mit TRIF, TBK1 und IRF3 gleich drei Gene, die typischerweise in der angeborenen Immunabwehr involviert sind, als proangiogene Faktoren [C.Korherr et al., PNAS, 103, 4240-4245, 2006].
Chien und Kollegen publizierten 2006 [Y.Chien et al., Cell 127, 157-170, 2006], dass TBK1-/- Zellen nur bedingt mit oncogenem Ras transformierbar sind, was eine Involvierung von TBK1 bei der Ras-vermittelten Transformation nahelegt. Desweiteren konnten Sie zeigen, dass ein RNAi vermittelter knock down von TBK1 Apoptose in MCF-7 und Panc-1 Zellen auslöst. Kürzlich publizierten Barbie und Kollegen, dass TBK1 in zahlreichen Krebszellinien mit mutierten K-Ras von essentieller Bedeutung ist, was nahelegt, dass eine TBK1 Intervention in entsprechenden Tumoren von therapeutischer Bedeutung sein könnte [D.A.Barbie et al., Nature Letters 1-5, 2009].

Durch Proteinkinasen hervorgerufene Erkrankungen sind durch eine anomale Aktivität oder Hyperaktivität solcher Proteinkinasen gekennzeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

Die wichtigsten Krebsarten, die unter Verwendung einer erfindungsgemäßen Verbindung behandelt werden können, umfassen Kolorektalkrebs, kleinzelligen Lungenkrebs, nicht-kleinzelligen Lungenkrebs, das multiple Myelom sowie das Nierenzellkarzinom und das Endometriumkarzinom, bersonders auch Krebsarten, in denen PTEN mutiert ist, u. a. Brustkrebs, Prostata-Krebs und Glioblastom.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemo -therapien und -bestrahlungen wiederherzustellen.

Unter Verbindungen der Formel I versteht man auch die Hydrate und Solvate dieser Verbindungen, ferner pharmazeutisch verwendbare Derivate.

Gegenstand der Erfindung sind auch die optisch aktiven Formen (Stereoisomeren), Salze, die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die Erfindung umfasst selbstverständlich auch die Solvate der Salze der erfindungsgemäßen Verbindungen.

Unter pharmazeutisch verwendbaren Derivaten versteht man z.B. die Salze der erfindungsgemäßen Verbindungen als auch sogenannte Prodrug-Verbindungen.

Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

> verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Salze, Tautomeren und Stereoisomeren, dadurch gekennzeichnet, daß man

in einer Masuda-Reaktion eine Verbindung der Formel II

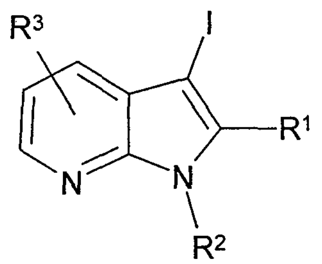

II

,

worin $R^1$ und $R^3$ die in Anspruch 1 angegebenen Bedeutungen haben und $R^2$ eine Aza-indol-Schutzgruppe bedeutet, mit 4,4,5,5-Tetramethyl-1,3,2-dioxaborolan umsetzt, und den intermediär entstehenden Boronsäure-pinacolester in einer Suzuki-Reaktion
mit einer Verbindung der Formel III

X-R III,

worin X Cl, Br oder I bedeutet,
und R die in Anspruch 1 angegebene Bedeutung hat,

umsetzt,
und/oder eine Base oder Säure der Formel I in eines ihrer Salze umwandelt.

Vor- und nachstehend haben die Reste R, $R^1$, $R^2$ und $R^3$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
In A können auch eine oder zwei CH- und/oder $CH_2$-Gruppen durch N, O- oder S-Atome und/oder durch -CH=CH-Gruppen ersetzt sein. So bedeutet A z.B. auch 2-Methoxy-ethyl oder 2-Hydroxyethyl.

A bedeutet weiterhin vorzugsweise unverzweigtes oder verzweigtes Alkyl mit 1-6 C-Atomen, worin auch 1-7 H-Atome durch F ersetzt sein können.

A' bedeutet vorzugsweise Methyl, Ethyl, Propyl, Isopropyl, Butyl, Isobutyl, sek.-Butyl oder tert.-Butyl, ferner Cyclopropyl, Cyclobutyl, Cyclopentyl, Cyclohexyl oder Cycloheptyl.

R bedeutet vorzugsweise unsubstituiertes oder einfach durch $R^5$ substituiertes 2, 7-Naphthyridin-4-yl.

$R^1$ bedeutet besonders bevorzugt H, Methyl, Ethyl oder Propyl.
$R^2$ bedeutet besonders bevorzugt H, Benzyl, Methyl, Ethyl oder Propyl.
$R^3$ bedeutet vorzugsweise H, A oder $-[C(R^6)_2]_n$-Het.
$R^3$ bedeutet besonders bevorzugt H, Methyl oder 1-Methyl-pyrazol-4-yl.

$R^5$ bedeutet vorzugsweise A, $-[C(R^6)_2]_n$-Ar, $OR^6$, OCyc, OHet', $N(R^6)_2$, =O oder $SO_2$Ar.
$R^5$ bedeutet ganz besonders bevorzugt Methyl, o-, m- oder p-Trifluormethylphenyl, Methoxy, Ethoxy, Propoxy oder Phenylsulfonyl.

$R^6$ bedeutet vorzugsweise H oder Methyl, besonders bevorzugt H.

Cyc bedeutet Cyclopropyl, Cyclobutyl, Cyclopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxy phenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl,
weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

Ar bedeutet vorzugsweise unsubstituiertes oder ein- oder zweifach durch A substituiertes Phenyl.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]-oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.

Die heterocyclischen Reste können auch teilweise oder vollständig hydriert sein.

Unsubstituiertes Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet weiterhin vorzugsweise einen unsubstituierten oder ein- oder zweifach durch A substituierten einkernigen aromatischen Heterocyclus mit 1 bis 4 N-, und/oder O- und/oder S-Atomen.

Het bedeutet ganz besonders bevorzugt unsubstituiertes oder ein- oder zweifach durch A substituiertes Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl oder Pyrazinyl.

Het' bedeutet vorzugsweise Piperidinyl, Pyrrolidinyl, Piperazinyl, Oxazolidinyl, Tetrahydropyranyl, Imidazolidinyl oder Morpholinyl, die ein- oder zweifach durch A und/oder =O (Carbonylsauerstoff) substituiert sein können.

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen und worin die nicht näher bezeichneten Reste die bei der Formel I angegebene Bedeutung haben, worin jedoch

in Ia

$R^2$ H, Benzyl oder A' bedeutet;

in Ib

$R^3$ H, A oder -[C($R^6$)$_2$]$_n$-Het bedeutet;

in Ic

Ar unsubstituiertes oder ein- oder zweifach durch A substituiertes Phenyl

bedeutet;

in Id

Het einen unsubstituierten oder ein- oder zweifach durch A substituierten einkernigen aromatischen Heterocyclus mit 1 bis 4 N-, und/oder O- und/oder S-Atomen

bedeutet;
in Ie

Het unsubstituiertes oder ein- oder zweifach durch A substituiertes Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl oder Pyrazinyl

bedeutet;
in If

Het' Piperidinyl, Pyrrolidinyl, Piperazinyl, Oxazolidinyl, Tetrahydropyranyl, Imidazolidinyl oder Morpholinyl, die ein- oder zweifach durch A und/oder =O (Carbonylsauerstoff) substituiert sein können,

bedeutet;
in Ig

A unverzweigtes oder verzweigtes Alkyl mit 1-6 C-Atomen, worin 1-7 H-Atome durch F ersetzt sein können

bedeutet;
in Ih

R unsubstituiertes oder ein- oder zweifach durch $R^5$ substituiertes

$R^1$ H oder A',
$R^2$ H, Benzyl oder A',
$R^3$ H, A oder $-[C(R^6)_2]_n$-Het,
$R^5$ A, Hal, CN, $-[C(R^6)_2]_n$-Ar, $-[C(R^6)_2]_n$-Het, $OR^6$, OCyc, OHet', $N(R^6)_2$, $SO_2A$, $SO_2Ar$ oder =O,
$R^6$ H oder A',
A unverzweigtes oder verzweigtes Alkyl mit 1-6 C-Atomen, worin 1-7 H-Atome durch F ersetzt sein können,
A' unverzweigtes oder verzweigtes Alkyl mit 1-4 C-Atomen oder Cyc,
Ar unsubstituiertes oder ein- oder zweifach durch A substituiertes Phenyl,
Het einen unsubstituierten oder ein- oder zweifach durch A substituierten einkernigen aromatischen Heterocyclus mit 1 bis 4 N-, O- und/oder S-Atomen,
Het' einen unsubstituierten oder ein- oder zweifach durch A und/oder =O substituierten einkernigen gesättigten Heterocyclus mit 1 oder 2 N-, O- und/oder S-Atomen,
Hal F, Cl, Br oder I,
n 0, 1 oder 2

bedeuten;
sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man in einer sequentiellen Masuda/Suzuki-Reaktion eine Verbindung der Formel II mit einer Verbindung der Formel III umsetzt.
In den Verbindungen der Formel III bedeutet X vorzugsweise Cl, Br oder I. Bei der Umsetzung der Verbindungen der Formel II mit den Verbindungen der Formel III erfolgt auch die Abspaltung der Aza-indol-schutzgruppe $R^2$, vorzugsweise eine tert.-Butyloxycarbonylgruppe.

Die Umsetzung erfolgt unter Bedingungen einer Suzuki-Kopplung.
Die Reaktionszeit liegt je nach den angewendeten Bedingungen zwischen einigen Minuten und 14 Tagen, die Reaktionstemperatur zwischen etwa -30° und 140°, normalerweise zwischen 0° und 110°, insbesondere zwischen etwa 70° und etwa 100°.
Als inerte Lösungsmittel eignen sich z.B. Kohlenwasserstoffe wie Hexan, Petrolether, Benzol, Toluol oder Xylol; chlorierte

Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist Dimethoxyethan, Methanol und/oder Dioxan.

Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$)Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasserals auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden.

Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Tautomeren und Stereoisomeren davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Tautomeren und Stereoisomeren davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff

zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z. B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandeln den Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, oben erwähnten Krankheitszustände geeignet sind.

Gegenstand der Erfindung sind ferner Arzneimittel enthaltend mindestens eine Verbindung der Formel I und/oder ihre pharmazeutisch verwendbaren Salze, Tautomeren und Stereoisomeren, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Salze, Tautomeren und Stereoisomere, einschließlich deren Mischungen in allen Verhältnissen,
und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Salze, Tautomeren und Stereoisomere, einschließlich deren Mischungen in allen Verhältnissen, und einer wirksamen Menge eines weiteren Arzneimittelwirkstoffs gelöst oder in lyophilisierter Form vorliegt.

**VERWENDUNG**

Die vorliegenden Verbindungen eignen sich als pharmazeutische Wirkstoffe für Säugetiere, insbesondere für den Menschen, bei der Behandlung und Bekämpfung von Krebserkrankungen.

Gegenstand der Erfindung sind weiterhin die Verbindungen der Formel I sowie ihrer pharmazeutisch verwendbaren Salze, Tautomeren und Stereoisomeren, einschließlich deren Mischungen in allen Verhältnissen, zur Verwendung zur Behandlung von Tumoren, Tumorwachstum, Tumormetastasen und/oder AIDS.

Gegenstand der Erfindung sind weiterhin die Verbindungen der Formel I sowie ihrer pharmazeutisch verwendbaren Salze, Tautomeren und Stereoisomeren, einschließlich deren Mischungen in allen Verhältnissen, zur Verwendung zur Behandlung von Fibrose, Restenose, HIV Infektion, Alzheimer, Atherosklerose und/oder zur Förderung der Wundheilung.

Die vorliegende Erfindung umfasst die Verwendung der Verbindungen der Formel I und/oder ihre physiologisch unbedenklichen Salze, Tautomeren und Stereoisomeren zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze, Tautomeren und Stereoisomeren zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung sind weiterhin die erfindungsgemäßen Verbindungen zur Verwendung in der Behandlung von Knochen-Pathologien, wobei die Knochenpathologie aus der Gruppe Osteosarkom, Osteoarthritis und Rachitis stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-ProteaseHemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]-phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.

"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat.

"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid.

"Zytotoxika" bezieht sich auf Verbindungen, die in erster Linie durch direkte Einwirkung auf die Zellfunktion zum Zelltod

führen oder die die Zellmyose hemmen oder diese stören, darunter Alkylierungsmittel, Tumornekrosefaktoren, interkalierende Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797. Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]-pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabinocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)-sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation/Vitalität von Tumorzellen *in vitro***

**1.0 Hintergrund**

In der vorliegenden Versuchsbeschreibung wird die Hemmung der Tumorzellproliferation/ Tumorzellvitalität durch Wirkstoffe beschrieben.
Die Zellen werden in geeigneter Zelldichte in Mikrotiterplatten (96-well Format) ausgesät und die Testsubstanzen werden in Form einer Konzentrationreihe zugegeben. Nach vier weiteren Tagen der Kultivierung in serumhaltigem Medium kann die Tumorzellproliferation/ Tumorzellvitalität mittels eines Alamarblue-Testsystem bestimmt werden.

**2.0 Versuchsdurchführung**

**2.1 Zellkultur**

Beispielsweise käuflich erhältliche Colon-Carcinom-Zelllinien, Zelllinien des Eierstocks, Zellinien der Prostata oder Zelllinien der Brust etc.
Die Zellen werden in Medium kultiviert. In Abständen von mehreren Tagen werden die Zellen mit Hilfe von Trypsin-Lösung von den Kulturschalen abgelöst und in geeigneten Verdünnung in frischem Medium ausgesät. Die Zellen werden bei 37° Celsius und 10% $CO_2$ kultiviert.

### 2.2. Aussaat der Zellen

Eine definierte Zellzahl (z.B. 2000 Zellen) werden pro Kultur/ well in einem Volumen von 180µl Kulturmedium in Mikrotiterplatten (96 well Zellkulturplatten) mit einer Mehrkanalpipette ausgesät. Die Zellen werden anschließend in einem CO2-Brutschrank (37°C und 10% CO2) kultiviert.

### 2.3. Zugabe der Testsubstanzen

Die Testsubstanzen werden beispielsweise in DMSO gelöst und anschließend in entsprechender Konzentration (gegebenenfalls einer Verdünnungsreihe) im Zellkulturmedium eingesetzt. Die Verdünnungs-stufen können je nach Effizienz der Wirkstoffe und gewünschter Spreizung der Konzentrationen angepasst werden. Die Testsubstanzen werden in entsprechenden Konzentrationen mit Zellkulturmedium versetzt. Die Zugabe der Testsubstanzen zu den Zellen kann am selben Tag wie die Aussat der Zellen erfolgen. Dazu wird aus der Vorverdünnungsplatte jeweils 20µl Substanzlösung in die Kulturen/wells gegeben. Die Zellen werden für weitere 4 Tage bei 37°Celsius und 10% $CO_2$ kultiviert.

### 2.4. Messung der Farbreaktion

Pro well werden jeweils 20µl AlamarBlue Reagenz gegeben und die Microtiterplatten werden beispielsweise für weitere sieben Stunden in einem CO2-Brutschrank (bei 37°C und 10% CO2) inkubiert. Die Platten werden an einem Reader mit einem Fluoreszenzfilter bei einer Wellenlänge von 540nm gemessen. Die Platten können direkt vor der Messung leicht geschüttelt werden.

### 3. Auswertung

Der Extinktionswert der Mediumkontrolle (keine Verwendung von Zellen und Testsubstanzen) wird von allen anderen Extinktionswerten subtrahiert. Die Kontrollen (Zellen ohne Testsubstanz) werden gleich 100 Prozent gesetzt und alle anderen Extinktionswerte hierzu in Beziehung gesetzt (beispielsweise in % der Kontrolle) ausgedrückt:

Rechnung:

$$\frac{100 * (\text{Wert mit Zellen und Testsubstanz - Wert der Mediumkontrolle})}{(\text{Wert mit Zellen - Wert der Mediumkontrolle})}$$

Die Bestimmung von $IC_{50}$ Werten (50%ige Hemmung) erfolgt mit Hilfe von Statistikprogrammen wie z.B. RS1.

### 4.0 Test zur Inhibierung von PDK1

Die Versuchsansätze werden in einem Flashplate-System mit 384 wells/Mikrotitrierplatte durchgeführt. Pro well werden jeweils die PDK1-Probe $His_6$-PDK1(□-50)( 3.4 nM), das PDK1-Substrat Biotin-bA-bA-KTFCGTPEYLAPEVRREP-RILSEEEQEMFRDFDYIADWC (400 nM), 4 µM ATP (mit 0.2µCi $^{33}$P-ATP/well) und die Testsubstanz in 50µl gebräuchlicher Versuchslösung für 60 Min bei 30°C inkubiert. Die Testsubstanzen werden in entsprechenden Konzentrationen (gegebenenfalls in einer Verdünnungsreihe) eingesetzt. Die Kontrolle wird ohne Testsubstanz durchgeführt. Die Reaktion wird mit gängigen Methoden gestoppt und gewaschen. Die Aktivität der Kinase wird über die eingebaute Radioaktivität im Topcount gemessen. Zur Bestimmung der unspezifischen Kinasereaktion (Leerwert) werden die Versuchsansätze in Gegenwart von 100 nM Staurosporin durchgeführt.

### 5.0 Auswertung

Die Radioaktivität (Zerfälle pro Minute) des Leerwerts (keine Verwendung von Testsubstanz in Gegenwart von Staurosporin) wird von allen anderen Radioaktivitätswerten substrahiert. Die Kontrollen (Kinaseaktivität ohne Testsubstanz) werden gleich 100 Prozent gesetzt und alle anderen Radioaktivitätswerte (nach Abzug des Leerwerts) hierzu in Beziehung gesetzt (beispielsweise in % der Kontrolle) ausgedrückt.

Rechnung:

$$\frac{100^{*}\ (\text{Wert der Kinaseaktivität mit Testsubstanz - Leerwert})}{(\text{Wert der Kontrolle - Leerwert})} = \%\ \text{der Kontrolle}$$

Die Bestimmung von $IC_{50}$ Werten (50%ige Hemmung) erfolgt mit Hilfe von Statistikprogrammen wie z.B. RS1. $IC_{50}$-Daten erfindungsgemäßer Verbindungen sind in Tabelle 1 angegeben.

| Material | Best. Nr. | Hersteller |
|---|---|---|
| Mikrotiterplatten für die Zellkultur (Nunclon Surface 96well Plate) | 167008 | Nunc |
| DMEM | P04-03550 | Pan Biotech |
| PBS (10x) Dulbecco | 14200-067 | Gibco |
| 96well Platten (Polypropylen) | 267334 | Nunc |
| AlamarBlue™ | BUF012B | Serotec |
| FCS | 1302 | Pan Biotech GmbH |
| Trypsin/EDTA Solution 10x | L 2153 | Biochrom AG |
| 75cm$^2$ Kulturflaschen | 353136 | BD Falcon |
| A2780 | 93112519 | ECACC |
| Colo205 | CCL222 | ATCC |
| MCF7 | HTB22 | ATCC |
| PC3 | CRL-1435 | ATCC |
| 384well Flash Platten | SMP410A001PK | Perkin Elmer |

APCI-MS (atmospheric pressure chemical ionization - mass spectrometry) $(M+H)^+$.

$IC_{50}$-Daten erfindungsgemäßer Verbindungen sind in Tabelle 1 angegeben.

IKKε - Kinase Test (IKKepsilon)

Der Kinaseassay wird als 384-well Flashplate assay durchgeführt.
1 nM IKKε, 800 nM biotinyliertes IεBα(19-42)-Peptid (Biotin-C6-C6-GLKKERLLDDRHDSGLDSMKDEE) und 10 µM ATP (mit 0,3 µCi $^{33}$P-ATP/well) werden in einem Gesamtvolumen von 50µl (10 mM MOPS, 10 mM Magnesiumacetat, 0,1 mM EGTA, 1 mM Dithiothreitol, 0,02 % Brij35, 0,1 % BSA, 0,1% BioStab, pH 7,5) ohne oder mit Prüfsubstanz für 120 Min bei 30°C inkubiert. Die Reaktion wird mit 25µl 200 mM EDTA-Lösung gestoppt, nach 30 Min bei Raumtemperatur abgesaugt und die Wells 3mal mit 100 µl 0,9%-ige NaCl-Lösung gewaschen. Der unspezifische Anteil der Kinasereaktion (Blank) wird mit 3 µM EMD 1126352 (BX-795) bestimmt. Radioaktivität wird im Topcount gemessen. $IC_{50}$-Werte werden mit RS1 berechnet.

TBK1 - Kinase Test

Der Kinaseassay wird als 384-well Flashplate assay durchgeführt.
0,6 nM TANK binding kinase (TBK1), 800 nM biotinyliertes MELK-derived peptide (Biotin-Ah-Ah-AKPKGNKDYHLQTCCGSLAYRRR) und 10 µM ATP (mit 0,25 µCi $^{33}$P-ATP/well) werden in einem Gesamtvolumen von 50µl (10 mM MOPS, 10 mM Magnesiumacetat, 0,1 mM EGTA, 1 mM DTT, 0,02 % Brij35, 0,1 % BSA, pH 7,5) ohne oder mit Prüfsubstanz für 120 Min bei 30°C inkubiert. Die Reaktion wird mit 25µl 200 mM EDTA-Lösung gestoppt, nach 30 Min bei Raumtemperatur abgesaugt und die Wells 3mal mit 100 µl 0,9%-ige NaCl-Lösung gewaschen. Der unspezifische Anteil der Kinasereaktion (Blank) wird mit 100 nM Staurosporine bestimmt. Radioaktivität wird im Topcount gemessen. $IC_{50}$-Werte werden mit RS1 berechnet.

In vitro-(Enzym-)Assay zur Bestimmung der Wirksamkeit der Inhibitoren der Hemmung von TGF-beta vermittelten Wirkungen

Als Beispiel wird die Fähigkeit der Inhibitoren zur Aufhebung der TGF-beta vermittelten Wachstumshemmung

getestet.

Zellen der Lungenepithelzellinie Mv1Lu werden in definierter Zelldichte in einer 96-well Mikrotiterplatte ausgesät und über Nacht unter Standardbedingungen kultiviert. Am Folgetag wird das Medium mit Medium, das 0,5%FCS und 1 ng/ml TGF-beta enthält, ersetzt und die Testsubstanzen in definierten Konzentrationen, in der Regel in Form von Verdünnungsreihen mit 5-fach Schritten, zugegeben. Die Konzentration des Lösungsmittels DMSO liegt konstant bei 0,5%. Nach zwei weiteren Tagen erfolgt Kristallviolett-Färbung der Zellen. Nach Extraktion des Kristallviolett aus den fixierten Zellen wird die Absorption bei 550 nm spektralphotometrisch gemessen. Sie kann als quantitatives Maß für die vorhandenen adhärenten Zellen und damit der Zellproliferation während der Kultur herangezogen werden.

HPLC/MS-Methode:

Säule: Chromolith SpeedROD RP-18e, 50 x 4.6 $mm^2$
Gradient: A:B = 96:4 to 0:100
Flussrate: 2.4 ml/min
Eluent A: Wasser + 0.05 % Ameisensäure
Eluent B: Acetonitril + 0.04 % Ameisensäure
Wellenlänge: 220 nm
Massenspektroskopie: Positiv-Modus
F. = Schmelzpunkt
MS (ESI): Massenspektroskopie (Elektrospray-Ionisation)
MS (EI): Massenspektroskopie (electron impact ionization)

<u>Herstellung von Zwischenstufen</u>

<u>Beispiel 1</u>

Herstellung von 3-Iod-5-(1-methyl-1H-pyrazol-4-yl)-pyrrolo[2,3-b]pyridin-1-carbonsäure-tert.-butylester

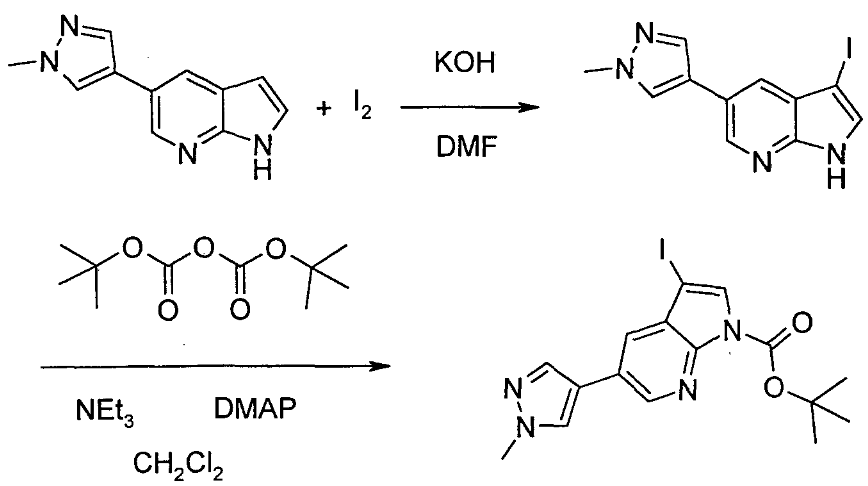


1.1 Zu einer Lösung von 4.00 g (20.2 mmol) 5-(1-Methyl-1 H-pyrazol-4-yl)-1 H-pyrrolo[2,3-b]pyridin in 60 ml DMF werden 2.80 g (49.9 mmol) festes Kaliumhydroxid gegeben und dann unter Rühren eine Lösung von 5.10 g (20.1 mmol) Iod in 40 ml DMF langsam zugetropft. Das Reaktionsgemisch wird mit Wasser und 300 mg Natriumdisulfit versetzt und mit Ethylacetat extrahiert. Die organische Phase wird über Natriumsulfat getrocknet und eingedampft: 3-Iod-5-(1-methyl-1H-pyrazol-4-yl)-1H-pyrrolo[2,3-b]pyridin als gelbliche Kristalle; HPLC/MS: 1.89 min, [M+H] 325.
1.2 Zu einer Suspension von 5.85 g (18.0 mmol) 3-Iod-5-(1-methyl-1 H-pyrazol-4-yl)-1H-pyrrolo[2,3-b]pyridin in 100 ml Dichlormethan werden 7.5 ml (54.1 mmol) Triethylamin und 220 mg (1.80 mmol) 4-(Dimethylamino)-pyridin gegeben. Dann wird langsam eine Lösung von 4.6 ml (21.5 mmol) Di-tert.-butyldicarbonat in 50 ml Dichlormethan zugetropft. Nach 4 Stunden Rühren bei Raumtemperatur wird das Reaktionsgemisch zwischen Wasser und Dichlormethan verteilt. Die organische Phase wird über Natriumsulfat getrocknet und im Vakuum eingedampft: 3-Iod-5-(1-methyl-1H-pyrazol-4-yl)-pyrrolo[2,3-b]pyridin-1-carbonsäure-tert-butylester als farblose Kristalle; HPLC/MS: 2.45 min, [M+H] 425.

Analog werden hergestellt:

3-Iod-pyrrolo[2,3-b]pyridin-1-carbonsäure-tert-butylester;
3-Iod-6-methyl-pyrrolo[2,3-b]pyridin-1-carbonsäure-tert-butylester;
3-Iod-2-methyl-pyrrolo[2,3-b]pyridin-1-carbonsäure-tert-butylester;
3-Iod-6-methoxy-pyrrolo[2,3-b]pyridin-1-carbonsäure-tert-butylester;
2-Cyclopropyl-3-iod-pyrrolo[2,3-b]pyridin-1-carbonsäure-tert-butylester;
3-Iod-6-methoxy-2-methyl-pyrrolo[2,3-b]pyridin-1-carbonsäure-tert-butylester;
3-Iod-2,6-dimethyl-pyrrolo[2,3-b]pyridin-1-carbonsäure-tert-butylester.

Herstellung von Verbindungen der Formel I

Beispiel 5

Herstellung von 2-Phenyl-5-(1H-pyrrolo[2,3-b]pyridin-3-yl)-[1,8]naphthyridin ("A5") (Vergleichsbeispiel)

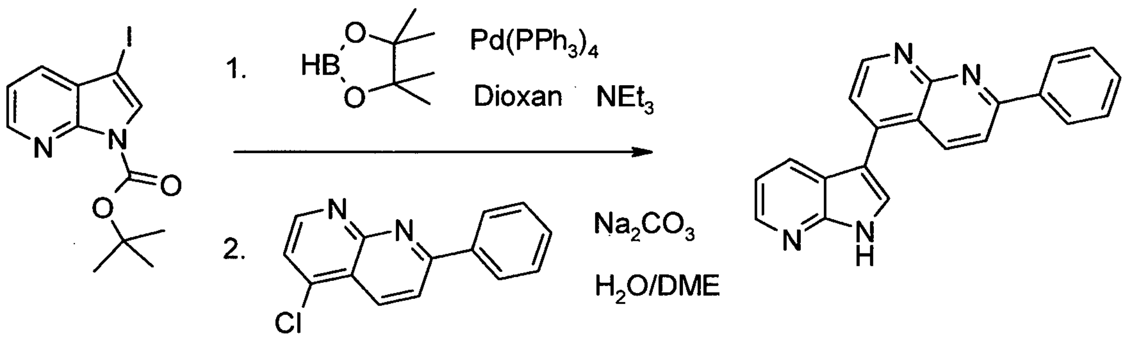


Ein Schlenkrohr wird unter Stickstoff nacheinander mit 189 mg (0.55 mmol) 3-Iod-pyrrolo[2,3-b]pyridin-1-carbonsäure-tert.-butylester, 9.5 mg (8.2 μmol) Tetrakis(triphenylphosphin)palladium, 2.3 ml Dioxan, 0.23 ml (1.6 mmol) Triethylamin und 80 μml (0.55 mmol) 4,4,5,5-Tetramethyl-1,3,2-dioxaborolan befüllt. Das Reaktionsgemisch wird 3 Stunden unter Stickstoff bei 80° C gerührt. Das Reaktionsgemisch wird auf Raumtemperatur gekühlt; 1 ml Wasser, 175 mg (2.1 mmol) Natriumcarbonat, 22 mg (19 μmol) Tetrakis-(triphenylphosphin)palladium, 132 mg (0.55 mmol) 5-Chlor-2-phenyl-1,8-naphthyridin und 4.6 ml 1,2-Dimethoxyethan werden unter Stickstoffatmosphäre zugefügt. Das Reaktionsgemisch wird 20 Stunden bei 100° C gerührt. Das Reaktionsgemisch wird auf Raumtemperatur gekühlt und im Vakuum eingedampft. Der Rückstand wird an einer Kieselgelsäule mit Methanol/Dichlormethan als Laufmittel chromatographiert: 2-Phenyl-5-(1 H-pyrrolo[2,3-b]pyridin-3-yl)-[1,8]naphthyridin ("A5") als gelbliche Kristalle; MS (ESI): 322 $[M+H]^+$; $^1$H-NMR ($d^6$-DMSO, 300 MHz): δ [ppm] = 12.40 (bs, 1H), 9.11 (d, J = 4.5 Hz, 1H), 8.65 (d, J = 8.8 Hz, 1 H), 8.41 - 8.32 (m, 3H), 8.25 (d, J = 8.8 Hz, 1 H), 8.06 (d, J = 2.5 Hz, 1 H), 8.01 (dd, J = 7.9 Hz, J = 1.5 Hz, 1 H), 7.70 (d, J = 4.5 Hz, 1 H), 7.65 - 7.51 (m, 3H), 7.20 (dd, J = 7.9 Hz, J = 4.7 Hz, 1 H).

Analog werden hergestellt:

4-(1H-Pyrrolo[2,3-b]pyridin-3-yl)-[2,7]naphthyridin-1-ylamin ("A24")

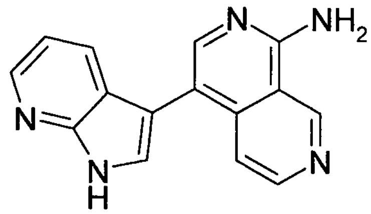

MS (ESI) = 262 [M+H]$^+$;

Beispiel 17

Herstellung von 2-(2-Hydroxyethyl)-4-(1H-pyrrolo[2,3-b]pyridin-3-yl)-2H-[2,7]naphthyridin-1-on ("A40")

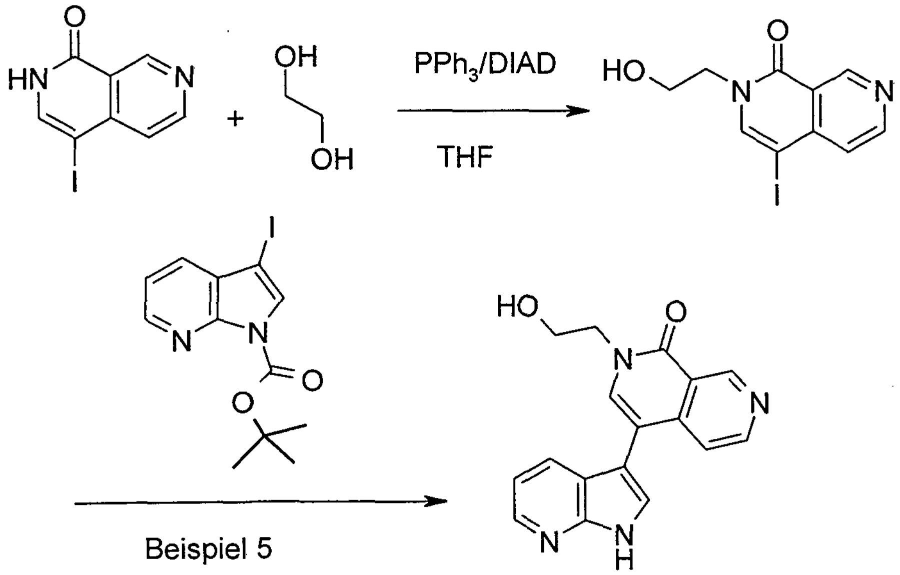


Eine Suspension von 2.11 g (7.74 mmol) 4-Iod-2H-[2,7]naphthyridin-1-on (zur Herstellung siehe A. Zhang et al, J. Comb. Chem. 9, Seite 916, 2007) und 3.08 g (11.6 mmol) Triphenylphosphin in 30 ml THF und 2.4 ml (77 mmol) Ethan-1,2-diol wird unter externer Eiskühlung mit 2.40 ml (11.6 mmol) Diisopropylazodicarboxylat versetzt. Die resultierende Lösung wird 3 Tage bei Raumtemperatur gerührt. Der entstandene Niederschlag wird abgesaugt, mit tert.-Butylmethylether gewaschen und im Vakuum getrocknet: 2-(2-Hydroxy-ethyl)-4-iod-2H-[2,7]naphthyridin-1-on als farbloser Feststoff; MS (ESI): 317 [M+H]$^+$;
$^1$H NMR (400 MHz, DMSO-$d_6$) $\delta$ [ppm] = 9.28 (d, *J*=0.6, 1H), 8.84 (d, *J*=5.6, 1H), 8.13 (s, 1H), 7.47 (dd, *J*=5.6, 0.7, 1H), 4.89 (s, 1H), 4.05 (t, *J*=5.4, 2H), 3.67 (t, *J*=5.4, 2H).

Analog zu Beispiel 5 wird 3-Iod-2-methyl-pyrrolo[2,3-b]pyridin-1-carbonsäure-tert.-butylester mit 2-(2-Hydroxyethyl)-4-iod-2H-[2,7]naphthyridin-1-on umgesetzt. Man erhält 2-(2-Hydroxyethyl)-4-(1 H-pyrrolo[2,3-b]pyridin-3-yl)-2H-[2,7]naphthyridin-1-on als farblosen Feststoff; MS (ESI): 307 [M+H]$^+$.

Inhibierung von PDK1, IKKepsilon, TBK1, TGF-beta

$IC_{50}$ von erfindungsgemäßen Verbindungen

| Verbindung Nr. | $IC_{50}$ [PDK1] | $IC_{50}$ [IKKepsilon] | $IC_{50}$ [TBK1] | $IC_{50}$ [TGF-beta] |
|---|---|---|---|---|
| "A24" | | | B | A |
| $IC_{50}$: 0.5 nM - 1 $\mu$M = A 1 $\mu$M - 10 $\mu$M = B | | | | |

Vitalitätsassay / $IC_{50}$ von erfindungsgemäßen Verbindungen

| Verbindung Nr. | $IC_{50}$ A2780 | $IC_{50}$ HCT 116 | |
|---|---|---|---|
| "A24" | B | B | |
| $IC_{50}$: 10 nM - 1 μM = A 1 μM - 10 μM = B | | | |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destillierten Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

**Patentansprüche**

**1.** Verbindungen der Formel I

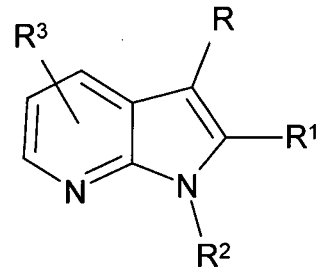

I

worin

R unsubstituiertes oder ein- oder zweifach durch $R^5$ substituiertes 2,7-Naphthyridin-4-yl,
$R^1$ H oder A',
$R^2$ H, A' oder $-[C(R^6)_2]n$-Ar,
$R^3$ H, A, Hai, CN, $-[C(R^6)_2]_n$-Ar, $-[C(R^6)_2]_n$-Het, $-[C(R^6)_2]_n$-Cyc, $OR^6$ oder $N(R^6)_2$,
$R^5$ A, Hal, CN, $-[C(R^6)_2]_n$-Ar, $-[C(R^6)_2]_n$-Het, $-[C(R^6)_2]_n$-Cyc, OCyc, OHet', $OR^6$, $N(R^6)_2$, $SO_2A$, $SO_2Ar$ oder =O,
$R^6$ H oder A',
A unverzweigtes oder verzweigtes Alkyl mit 1-6 C-Atomen, worin eine oder zwei $CH_2$-Gruppen durch O-, N und/oder S-Atome und/oder durch -CH=CH-Gruppen und/oder auch 1-7 H-Atome durch F ersetzt sein können,
A' unverzweigtes oder verzweigtes Alkyl mit 1-4 C-Atomen oder Cyc,
Cyc Cycloalkyl mit 3, 4, 5, 6 oder 7 C-Atomen,
Ar unsubstituiertes oder ein-, zwei- oder dreifach durch Hai, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2N(R^6)_2$ und/oder $S(O)_nA$ substituiertes Phenyl,
Het einen unsubstituierten oder ein- oder zweifach durch Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2NR^6$ und/oder $S(O)_nA$ substituierten ein- oder zweikernigen gesättigten, ungesättigten oder aromatischen Heterocyclus mit 1 bis 4 N-, O- und/oder S-Atomen,
Het' einen unsubstituierten oder ein- oder zweifach durch A und/oder =O substituierten einkernigen gesättigten Heterocyclus mit 1 oder 2 N-, O- und/oder S-Atomen,
Hal F, Cl, Br oder I,
n 0, 1 oder 2

bedeuten,
sowie ihre pharmazeutisch verwendbaren Salze, Tautomere und Stereoisomere, einschließlich deren Mischungen in allen Verhältnissen.

**2.** Verbindungen nach Anspruch 1, worin

$R^2$ H, Benzyl oder A'

bedeutet,
sowie ihre pharmazeutisch verwendbaren Salze, Tautomere und Stereoisomere, einschließlich deren Mischungen in allen Verhältnissen.

**3.** Verbindungen nach Anspruch 1 oder 2, worin

$R^3$ H, A oder $-[C(R^6)_2]_n$-Het

bedeutet, sowie ihre pharmazeutisch verwendbaren Salze, Tautomere und Stereoisomere, einschließlich deren Mischungen in allen Verhältnissen.

**4.** Verbindungen nach einem oder mehreren der Ansprüche 1-3, worin

Ar unsubstituiertes oder ein- oder zweifach durch A substituiertes Phenyl

bedeutet,
sowie ihre pharmazeutisch verwendbaren Salze, Tautomere und Stereoisomere, einschließlich deren Mischungen in allen Verhältnissen.

**5.** Verbindungen nach einem oder mehreren der Ansprüche 1-4, worin

Het einen unsubstituierten oder ein- oder zweifach durch A substituierten einkernigen aromatischen Heterocyclus mit 1 bis 4 N-, O- und/oder S-Atomen, bevorzugt unsubstituiertes oder ein- oder zweifach durch A substituiertes Furyl, Thienyl, Pyrrolyl, Imidazolyl, Pyrazolyl, Oxazolyl, Isoxazolyl, Thiazolyl, Pyridyl, Pyrimidinyl, Triazolyl, Tetrazolyl, Thiadiazol, Pyridazinyl oder Pyrazinyl,

bedeutet,
sowie ihre pharmazeutisch verwendbaren Salze, Tautomere und Stereoisomere, einschließlich deren Mischungen in allen Verhältnissen.

**6.** Verbindungen nach einem oder mehreren der Ansprüche 1-5, worin

Het' Piperidinyl, Pyrrolidinyl, Piperazinyl, Oxazolidinyl, Tetrahydropyranyl, Imidazolidinyl oder Morpholinyl, die ein- oder zweifach durch A und/oder =O (Carbonylsauerstoff) substituiert sein können,

bedeutet,
sowie ihre pharmazeutisch verwendbaren Salze, Tautomere und Stereoisomere, einschließlich deren Mischungen in allen Verhältnissen.

**7.** Verbindungen nach einem oder mehreren der Ansprüche 1-6, worin

A unverzweigtes oder verzweigtes Alkyl mit 1-6 C-Atomen, worin 1-7 H-Atome durch F ersetzt sein können,

bedeutet, sowie ihre pharmazeutisch verwendbaren Salze, Tautomere und Stereoisomere, einschließlich deren Mischungen in allen Verhältnissen.

**8.** Verbindungen nach einem oder mehreren der Ansprüche 1-7, worin

R unsubstituiertes oder ein- oder zweifach durch $R^5$ substituiertes 2,7-Naphthyridin-4-yl sein kann,
$R^1$ H oder A',
$R^2$ H, Benzyl oder A',
$R^3$ H, A oder $-[C(R^6)_2]_n$-Het,
$R^5$ A, Hal, CN, $-[C(R^6)_2]_n$-Ar, $-[C(R^6)_2]_n$-Het, $OR^6$, OCyc, OHet', $N(R^6)_2$, $SO_2A$, $SO_2Ar$ oder =O,
$R^6$ H oder A',
A unverzweigtes oder verzweigtes Alkyl mit 1-6 C-Atomen, worin 1-7 H-Atome durch F ersetzt sein können,
A' unverzweigtes oder verzweigtes Alkyl mit 1-4 C-Atomen oder Cyc,
Cyc Cycloalkyl mit 3, 4, 5, 6 oder 7 C-Atomen,
Ar unsubstituiertes oder ein- oder zweifach durch A substituiertes Phenyl,
Het einen unsubstituierten oder ein- oder zweifach durch A substituierten einkernigen aromatischen Heterocyclus mit 1 bis 4 N-, O- und/oder S-Atomen,
Het' einen unsubstituierten oder ein- oder zweifach durch A und/oder =O substituierten einkernigen gesättigten Heterocyclus mit 1 oder 2 N-, O- und/oder S-Atomen,
Hal F, Cl, Br oder I,
n 0, 1 oder 2

bedeuten, sowie ihre pharmazeutisch verwendbaren Salze, Tautomere und Stereoisomere, einschließlich deren Mischungen in allen Verhältnissen.

**9.** Verbindungen nach Anspruch 1, ausgewählt aus der Gruppe

| Verbindung Nr. | Name und/oder Struktur |
|---|---|
| "A24" | 4-(1H-Pyrrolo[2,3-b]pyridin-3-yl)-[2,7]naphthyridin-1-ylamin |
| "A40" | 2-(2-Hydroxyethyl)-4-(1H-pyrrolo[2,3-b]pyridin-3-yl)-2H-[2,7]naphthyridin-1-on |

sowie ihre pharmazeutisch verwendbaren Salze, Tautomere und Stereoisomere, einschließlich deren Mischungen

in allen Verhältnissen.

**10.** Verfahren zur Herstellung von Verbindungen der Formel I nach den Ansprüchen 1-9 sowie ihrer pharmazeutisch verwendbaren Salze, Tautomeren und Stereoisomeren, **dadurch gekennzeichnet, daß** man in einer Masuda-Reaktion eine Verbindung der Formel II

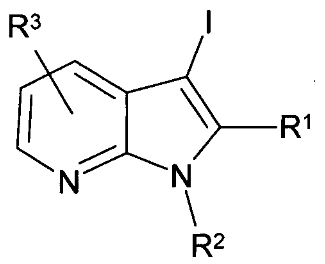

II

,

worin $R^1$ und $R^3$ die in Anspruch 1 angegebenen Bedeutungen haben
und $R^2$ eine Aza-indol-Schutzgruppe bedeutet,

mit 4,4,5,5-Tetramethyl-1,3,2-dioxaborolan umsetzt, und den intermediär entstehenden Boronsäure-pinacolester in einer Suzuki-Reaktion
mit einer Verbindung der Formel III

X-R III,

worin X Cl, Br oder I bedeutet,
und R die in Anspruch 1 angegebene Bedeutung hat,

umsetzt,
und/oder
eine Base oder Säure der Formel I in eines ihrer Salze umwandelt.

**11.** Arzneimittel, enthaltend mindestens eine Verbindung der Formel I gemäß Anspruch 1-9 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

**12.** Verbindungen der Formel I gemäß Anspruch 1-9 sowie ihrer pharmazeutisch verwendbaren Salze, Tautomeren und Stereoisomeren, einschließlich deren Mischungen in allen Verhältnissen, zur Verwendung zur Behandlung von Tumoren, Tumorwachstum, Tumormetastasen und/oder AIDS.

**13.** Verbindungen der Formel I gemäß Anspruch 1-9 und/oder ihrer physiologisch unbedenklichen Salze, Tautomeren und Stereoisomeren zur Verwendung zur Behandlung von Tumoren, wobei eine therapeutisch wirksame Menge einer Verbindung der Formel I' in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

**14.** Verbindungen der Formel I gemäß Anspruch 1-9 und/oder ihrer physiologisch unbedenklichen Salze, Tautomeren und Stereoisomeren zur Verwendung zur Behandlung von Tumoren, wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

**1.** Compounds of the formula I

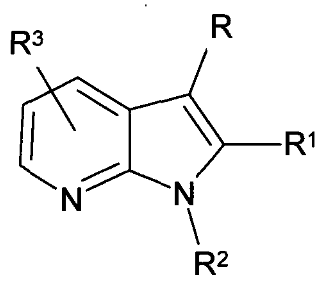

I

in which

R denotes 2,7-naphthyridin-1-yl which is unsubstituted or mono- or disubstituted by $R^5$;
$R^1$ denotes H or A',
$R^2$ denotes H, A' or $-[C(R^6)_2]_n$-Ar,
$R^3$ denotes H, A, Hal, CN, $-[C(R^6)_2]_n$-Ar, $-[C(R^6)_2]_n$-Het, $-[C(R^6)_2]_n$-Cyc, $OR^6$ or $N(R^6)_2$,
$R^5$ denotes A, Hal, CN, $-[C(R^6)_2]_n$-Ar, $-[C(R^6)_2]_n$-Het, $-[C(R^6)_2]_n$-Cyc, OCyc, OHet', $OR^6$, $N(R^6)_2$, $SO_2A$, $SO_2Ar$ or =O,
$R^6$ denotes H or A',
A denotes unbranched or branched alkyl having 1-6 C atoms, in which one or two $CH_2$ groups may be replaced by O, N and/or S atoms and/or by -CH=CH- groups and/or, in addition, 1-7 H atoms may be replaced by F,
A' denotes unbranched or branched alkyl having 1-4 C atoms or Cyc,
Cyc denotes cycloalkyl having 3, 4, 5, 6 or 7 C atoms,
Ar denotes phenyl which is unsubstituted or mono-, di- or trisubstituted by Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2N(R^6)_2$ and/or $S(O)_nA$,
Het denotes a mono- or bicyclic saturated, unsaturated or aromatic heterocycle having 1 to 4 N, O and/or S atoms which is unsubstituted or mono- or disubstituted by Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2NR^6$ and/or $S(O)_nA$,
Het' denotes a monocyclic saturated heterocycle having 1 or 2 N, O and/or S atoms which is unsubstituted or mono- or disubstituted by A and/or =O,
Hal denotes F, Cl, Br or I,
n denotes 0, 1 or 2,

and pharmaceutically usable salts, tautomers and stereoisomers thereof, including mixtures thereof in all ratios.

**2.** Compounds according to Claim 1 in which

$R^2$ denotes H, benzyl or A',

and pharmaceutically usable salts, tautomers and stereoisomers thereof, including mixtures thereof in all ratios.

**3.** Compounds according to Claim 1 or 2 in which

$R^3$ denotes H, A or $-[C(R^6)_2]_n$-Het,

and pharmaceutically usable salts, tautomers and stereoisomers thereof, including mixtures thereof in all ratios.

**4.** Compounds according to one or more of Claims 1-3 in which

Ar denotes phenyl which is unsubstituted or mono- or disubstituted by A,

and pharmaceutically usable salts, tautomers and stereoisomers thereof, including mixtures thereof in all ratios.

**5.** Compounds according to one or more of Claims 1-4 in which

Het denotes a monocyclic aromatic heterocycle having 1 to 4 N, O and/or S atoms which is unsubstituted or mono- or disubstituted by A, preferably furyl, thienyl, pyrrolyl, imidazolyl, pyrazolyl, oxazolyl, isoxazolyl, thiazolyl, pyridyl, pyrimidinyl, triazolyl, tetrazolyl, thiadiazole, pyridazinyl or pyrazinyl, each of which is unsubstituted or

mono- or disubstituted by A,

and pharmaceutically usable salts, tautomers and stereoisomers thereof, including mixtures thereof in all ratios.

**6.** Compounds according to one or more of Claims 1-5 in which

Het' denotes piperidinyl, pyrrolidinyl, piperazinyl, oxazolidinyl, tetrahydropyranyl, imidazolidinyl or morpholinyl, each of which may be mono- or disubstituted by A and/or =O (carbonyl oxygen),

and pharmaceutically usable salts, tautomers and stereoisomers thereof, including mixtures thereof in all ratios.

**7.** Compounds according to one or more of Claims 1-6 in which

A denotes unbranched or branched alkyl having 1-6 C atoms, in which 1-7 H atoms may be replaced by F,

and pharmaceutically usable salts, tautomers and stereoisomers thereof, including mixtures thereof in all ratios.

**8.** Compounds according to one or more of Claims 1-7 in which

R denotes 2,7-naphthyridin-1-yl which is unsubstituted or mono- or disubstituted by $R^5$;
$R^1$ denotes H or A',
$R^2$ denotes H, benzyl or A',
$R^3$ denotes H, A or $-[C(R^6)_2]_n$-Het,
$R^5$ denotes A, Hal, CN, $-[C(R^6)_2]_n$-Ar, $-[C(R^6)_2]_n$-Het, $OR^6$, OCyc, OHet', $N(R^6)_2$, $SO_2A$, $SO_2Ar$ or =O,
$R^6$ denotes H or A',
A denotes unbranched or branched alkyl having 1-6 C atoms, in which 1-7 H atoms may be replaced by F,
A' denotes unbranched or branched alkyl having 1-4 C atoms or Cyc,
Cyc denotes cycloalkyl having 3, 4, 5, 6 or 7 C atoms,
Ar denotes phenyl which is unsubstituted or mono- or disubstituted by A,
Het denotes a monocyclic aromatic heterocycle having 1 to 4 N, O and/or S atoms which is unsubstituted or mono- or disubstituted by A,
Het' denotes a monocyclic saturated heterocycle having 1 or 2 N, O and/or S atoms which is unsubstituted or mono- or disubstituted by A and/or =O,
Hal denotes F, Cl, Br or I,
n denotes 0, 1 or 2,

and pharmaceutically usable salts, tautomers and stereoisomers thereof, including mixtures thereof in all ratios.

**9.** Compounds according to Claim 1, selected from the group

| Compound No. | Name and/or structure |
|---|---|
| "A24" | 4-(1H-Pyrrolo[2,3-b]pyridin-3-yl)-2,7-naphthyridin-1-yl-amine |
| "A40" | 2-(2-Hydroxyethyl)-4-(1H-pyrrolo[2,3-b]pyridin-3-yl)-2H-2,7-naphthyridin-1-one |

and pharmaceutically usable salts, tautomers and stereoisomers thereof, including mixtures thereof in all ratios.

**10.** Process for the preparation of compounds of the formula I according to Claims 1-9 and pharmaceutically usable salts, tautomers and stereoisomers thereof, **characterised in that** a compound of the formula II

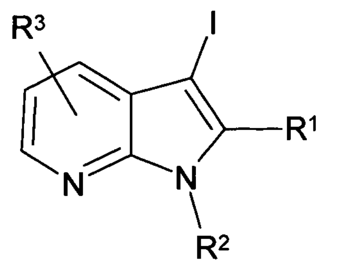

II

,

in which $R^1$ and $R^3$ have the meanings indicated in Claim 1
and $R^2$ denotes an azaindole-protecting group,

is reacted with 4,4,5,5-tetramethyl-1,3,2-dioxaborolane in a Masuda reaction, and the boronic acid pinacole ester formed as intermediate is reacted
with a compound of the formula III

X-R III,

in which X denotes Cl, Br or I,
and R has the meaning indicated in Claim 1,

in a Suzuki reaction,
and/or
a base or acid of the formula I is converted into one of its salts.

**11.** Medicaments comprising at least one compound of the formula I according to Claims 1-9 and/or pharmaceutically usable salts, tautomers and stereoisomers thereof, including mixtures thereof in all ratios, and optionally excipients and/or adjuvants.

**12.** Compounds of the formula I according to Claims 1-9 and pharmaceutically usable salts, tautomers and stereoisomers thereof, including mixtures thereof in all ratios, for use for the treatment of tumours, tumour growth, tumour metastases and/or AIDS.

**13.** Compounds of the formula I according to Claims 1-9 and/or physiologically acceptable salts, tautomers and stereoisomers thereof for use for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**14.** Compounds of the formula I according to Claims 1-9 and/or physiologically acceptable salts, tautomers and stereoisomers thereof for use for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

**1.** Composés de formule I

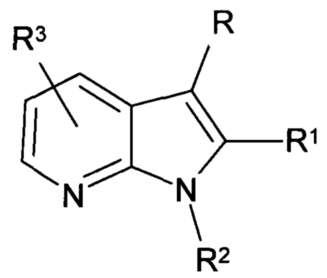

I

dans lesquels

R désigne 2,7-naphtyridin-1-yle qui est non substitué ou mono- ou disubstitué par $R^5$;
$R^1$ désigne H ou A',
$R^2$ désigne H, A' ou $-[C(R^6)_2]_n$-Ar,
$R^3$ désigne H, A, Hal, CN, $-[C(R^6)_2]_n$-Ar, $-[C(R^6)_2]_n$-Hét, $-[C(R^6)_2]_n$-Cyc, $OR^6$ ou $N(R^6)_2$,
$R^5$ désigne A, Hal, CN, $-[C(R^6)_2]_n$-Ar, $-[C(R^6)_2]_n$-Hét, $-[C(R^6)_2]_n$-Cyc, OCyc, OHét', $OR^6$, $N(R^6)_2$, $SO_2A$, $SO_2Ar$ ou =O,
$R^6$ désigne H ou A',
A désigne alkyle ramifié ou non ramifié ayant 1-6 atomes de C, où un ou deux groupements $CH_2$ peuvent être remplacés par des atomes de O, N et/ou S et/ou par des groupements -CH=CH- et/ou, en outre, 1-7 atomes de H peuvent être remplacés par F,
A' désigne alkyle ramifié ou non ramifié ayant 1-4 atomes de C ou Cyc,
Cyc désigne cycloalkyle ayant 3, 4, 5, 6 ou 7 atomes de C,
Ar désigne phényle qui est non substitué ou mono-, di- ou trisubstitué par Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2N(R^6)_2$ et/ou $S(O)_nA$,
Hét désigne un hétérocycle saturé, insaturé ou aromatique hétérocyclique mono- ou bicyclique ayant de 1 à 4 atomes de N, O et/ou S qui est non substitué ou mono- ou disubstitué par Hal, A, $-[C(R^6)_2]_nOR^6$, $N(R^6)_2$, $NO_2$, CN, $COOR^6$, $CON(R^6)_2$, $NR^6COA$, $NR^6SO_2A$, $COR^6$, $SO_2NR^6$ et/ou $S(O)_nA$,
Hét' désigne un hétérocycle saturé monocyclique ayant 1 ou 2 atomes de N, O et/ou S qui est non substitué ou mono- ou disubstitué par A et/ou =O,
Hal désigne F, Cl, Br ou I,
n désigne 0, 1 ou 2,

ainsi que les sels, tautomères et stéréoisomères pharmaceutiquement utilisables de ceux-ci, y compris des mélanges de ceux-ci en toutes proportions.

2. Composés selon la revendication 1, dans lesquels

$R^2$ désigne H, benzyle ou A',

ainsi que les sels, tautomères et stéréoisomères pharmaceutiquement utilisables de ceux-ci, y compris des mélanges de ceux-ci en toutes proportions.

3. Composés selon la revendication 1 ou 2, dans lesquels

$R^3$ désigne H, A ou $-[C(R^6)_2]_n$-Hét,

ainsi que les sels, tautomères et stéréoisomères pharmaceutiquement utilisables de ceux-ci, y compris des mélanges de ceux-ci en toutes proportions.

4. Composés selon l'une ou plusieurs parmi les revendications 1-3, dans lesquels

Ar désigne phényle qui est non substitué ou mono- ou disubstitué par A,

ainsi que les sels, tautomères et stéréoisomères pharmaceutiquement utilisables de ceux-ci, y compris des mélanges de ceux-ci en toutes proportions.

**5.** Composés selon l'une ou plusieurs parmi les revendications 1-4, dans lesquels

Hét désigne un hétérocycle monocyclique aromatique ayant de 1 à 4 atomes de N, O et/ou S qui est non substitué ou mono- ou disubstitué par A, de préférence furyle, thiényle, pyrrolyle, imidazolyle, pyrazolyle, oxazolyle, isoxazolyle, thiazolyle, pyridinyle, pyrimidinyle, triazolyle, tétrazolyle, thiadiazole, pyridazinyle ou pyrazinyle, chacun parmi lesquels étant non substitué ou mono- ou disubstitué par A,

ainsi que les sels, tautomères et stéréoisomères pharmaceutiquement utilisables de ceux-ci, y compris des mélanges de ceux-ci en toutes proportions.

**6.** Composés selon l'une ou plusieurs parmi les revendications 1-5, dans lesquels

Hét' désigne pipéridinyle, pyrrolidinyle, pipérazinyle, oxazolidinyle, tétrahydropyranyle, imidazolidinyle ou morpholinyle, chacun parmi lesquels pouvant être mono- ou disubstitué par A et/ou =O (oxygène du carbonyle),

ainsi que les sels, tautomères et stéréoisomères pharmaceutiquement utilisables de ceux-ci, y compris des mélanges de ceux-ci en toutes proportions.

**7.** Composés selon l'une ou plusieurs parmi les revendications 1-6, dans lesquels

A désigne alkyle ramifié ou non ramifié ayant 1-6 atomes de C, où 1-7 atomes de H peuvent être remplacés par F,

ainsi que les sels, tautomères et stéréoisomères pharmaceutiquement utilisables de ceux-ci, y compris des mélanges de ceux-ci en toutes proportions.

**8.** Composés selon l'une ou plusieurs parmi les revendications 1-7, dans lesquels

R désigne 2,7-naphtyridin-1-yle qui est non substitué ou mono- ou disubstitué par $R^5$;
$R^1$ désigne H ou A',
$R^2$ désigne H, benzyle ou A',
$R^3$ désigne H, A ou $-[C(R^6)_2]_n$-Hét,
$R^5$ désigne A, Hal, CN, $-[C(R^6)_2]_n$-Ar, $-[C(R^6)_2]_n$-Hét, $OR^6$, OCyc, OHét', $N(R^6)_2$, $SO_2A$, $SO_2Ar$ ou =O,
$R^6$ désigne H ou A',
A désigne alkyle ramifié ou non ramifié ayant 1-6 atomes de C, où 1-7 atomes de H peuvent être remplacés par F,
A' désigne alkyle ramifié ou non ramifié ayant 1-4 atomes de C ou Cyc,
Cyc désigne cycloalkyle ayant 3, 4, 5, 6 ou 7 atomes de C,
Ar désigne phényle qui est non substitué ou mono- ou disubstitué par A,
Hét désigne hétérocycle monocyclique aromatique ayant de 1 à 4 atomes de N, O et/ou S qui est non substitué ou monoou disubstitué par A,
Hét' désigne hétérocycle monocyclique aromatique ayant 1 ou 2 atomes de N, O et/ou S qui est non substitué ou mono- ou disubstitué par A et/ou =O,
Hal désigne F, Cl, Br ou I,
n désigne 0, 1 ou 2,

ainsi que les sels, tautomères et stéréoisomères pharmaceutiquement utilisables de ceux-ci, y compris des mélanges de ceux-ci en toutes proportions.

**9.** Composés selon la revendication 1, choisis dans le groupe constitué par

| Composé n° | Nom et/ou structure |
|---|---|
| « A24 » | 4-(1H-Pyrrolo[2,3-b]pyridin-3-yl)-2,7-naphtyridin-1-yl-amine |
| « A40 » | 2-(2-Hydroxyéthyl)-4-(1H-pyrrolo[2,3-b]pyridin-3-yl)-2H-2,7-naphtyridin-1-one |

ainsi que les sels, tautomères et stéréoisomères pharmaceutiquement utilisables de ceux-ci, y compris des mélanges de ceux-ci en toutes proportions.

**10.** Procédé de préparation de composés de formule I selon les revendications 1-9 ainsi que de sels, tautomères et stéréoisomères pharmaceutiquement utilisables de ceux-ci, **caractérisé en ce qu'**un composé de formule II

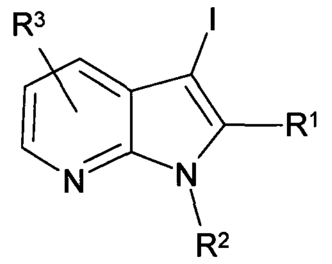

II

,

dans laquelle $R^1$ et $R^3$ revêtent les significations indiquées selon la revendication 1
et $R^2$ désigne un groupement protecteur d'azaindole,

est réagi avec du 4,4,5,5-tétraméthyl-1,3,2-dioxaborolane dans une réaction de Masuda, et l'ester de pinacol de l'acide boronique formé comme intermédiaire est réagi
avec un composé de formule III

X-R III,

dans laquelle X désigne Cl, Br ou I,
et R revêt la signification indiqué selon la revendication 1,

dans une réaction de Suzuki,
et/ou
une base ou un acide de formule I est converti(e) en l'un de ses sels.

**11.** Médicaments comprenant au moins un composé de formule I selon les revendications 1-9 et/ou des sels, tautomères et stéréoisomères pharmaceutiquement utilisables de celui-ci, y compris des mélanges de ceux-ci en toutes proportions, et éventuellement des excipients et/ou des adjuvants.

**12.** Composés de formule I selon les revendications 1-9 ainsi que les sels, tautomères et stéréoisomères pharmaceutiquement utilisables de ceux-ci, y compris des mélanges de ceux-ci en toutes proportions, pour une utilisation dans le traitement de tumeurs, de la croissance tumorale, des métastases tumorales et/ou du SIDA.

**13.** Composés de formule I selon les revendications 1-9 et/ou des sels, tautomères et stéréoisomères physiologiquement acceptables de ceux-ci, pour une utilisation dans le traitement de tumeurs, où une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**14.** Composés de formule I selon les revendications 1-9 et/ou des sels, tautomères et stéréoisomères physiologiquement acceptables de ceux-ci, pour une utilisation dans le traitement de tumeurs, où une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005095400 A1
- WO 2008079988 A2
- WO 2008112217 A1
- WO 2008005457 A
- WO 2008124849 A
- WO 2006106326 A1
- WO 2008156726 A1
- WO 2009054941 A1
- JP 2007099640 A
- US 20070203142 A1
- WO 2012000595 A1
- WO 0050032 A
- US 6069134 A


### In der Beschreibung aufgeführte Nicht-Patentliteratur


- **J.S. BOEHM et al.** *Cell,* 2007, vol. 129, 1065-1079
- **S.F.EDDY et al.** *Cancer Res.,* 2005, vol. 65 (24), 11375-11383
- **C.KORHERR et al.** *PNAS,* 2006, vol. 103, 4240-4245
- **Y.CHIEN et al.** *Cell,* 2006, vol. 127, 157-170
- **D.A.BARBIE et al.** *Nature Letters,* 2009, 1-5
- *Int. J. Pharm.,* 1995, vol. 115, 61-67
- *Pharmaceutical Research,* 1986, vol. 3 (6), 318
- **A. ZHANG et al.** *J. Comb. Chem.,* 2007, vol. 9, 916